# EUROPEAN PATENT SPECIFICATION

(11) **EP 3 971 744 B1**
(45) Date of publication and mention of the grant of the patent: **26.02.2025**
(21) Application number: 20827327.6
(22) Date of filing: 23.03.2020
(51) Int. Cl.: G06F 21/32, G06V 40/13, G06T 1/00, A61B 5/1172, H04L 9/40

(54) **BIOMETRIC AUTHENTICATION DEVICE**
VORRICHTUNG ZUR BIOMETRISCHEN AUTHENTIFIZIERUNG
DISPOSITIF D'AUTHENTIFICATION BIOMÉTRIQUE

(30) Priority: 17.06.2019 JP 2019112025
(43) Date of publication of application: 23.03.2022
(73) Proprietor: KABUSHIKI KAISHA TOSHIBA, Minato-ku Tokyo 105-0023 (JP); Toshiba Infrastructure Systems & Solutions Corporation, Kawasaki-shi, Kanagawa 212-0013 (JP)
(72) Inventor: FUKUOKA, Hiroki, Kawasaki-shi, Kanagawa 212-0013 (JP); TOSHIMITSU, Kiyoshi, Kawasaki-shi, Kanagawa 212-0013 (JP)
(74) Representative: Horn Kleimann Waitzhofer Schmid-Dreyer Patent- und Rechtsanwälte PartG mbB
(86) International application number: PCT/JP2020/012690
(87) International publication number: WO 2020/255510

(56) References cited:
- EP-A1- 1 775 674
- EP-A1- 3 608 808
- WO-A1-2018/195976
- JP-A- 2000 057 328
- JP-A- 2002 157 586
- JP-A- 2009 032 227
- JP-A- 2019 074 908
- US-B1- 6 522 773

## Description

Embodiments described herein relate generally to a biometric authentication device.

### BACKGROUND

Conventionally, there has been known a technique in which a biometric authentication sensor is incorporated into hardware such as an automatic teller machine (ATM) of a bank, an entrance and exit management terminal, a personal computer, and a mobile phone terminal, and the biometric authentication sensor performs biometric authentication of a user.

In recent years, miniaturization of biometric authentication devices has progressed, and it is conceivable for a biometric authentication sensor to be mounted on a portable medium having a size such that it can be held by one hand. Further, such a biometric authentication device is equipped with a rechargeable battery. However, as the biometric authentication device becomes smaller, the battery that can be mounted becomes smaller. Therefore, the biometric authentication device has a problem with power saving.

For example, when a biometric authentication device is used, a user may manually operate a power switch to turn on the biometric authentication device and then operate the power switch to turn off the biometric authentication device after fingerprint authentication is completed. However, this forces the user to use the biometric authentication device in a complicated manner.

EP 1 775 674 A1 discloses press-trigger fingerprint sensor module comprising two embedded switches, a sensor panel and a fingerprint recognition processor. When the user puts the finger in predetermined position over the sensor panel, the first switch is activated. The first switch sends out a high signal to have power connected to the fingerprint sensor. Without changing the position of the finger, the pressure being exerted perpendicular to the panel surface is transferred to the second switch, which then senses the presence of the finger and captures an image. The fingerprint recognition is performed subsequently.

Document WO 2018/195976 A1 discloses a fingerprint identification-based boot method and apparatus. The method comprises: upon detection of a boot signal, acquiring fingerprint data; matching the fingerprint data against pre-stored predetermined fingerprint data; and if the matching is successful, then logging on to a terminal apparatus. The fingerprint identification-based boot method and apparatus provided by the present application improve efficiency in logging on to a terminal apparatus, and increase a battery run-time of the terminal apparatus.

US 6 522 773 B1 discloses a sensor with a biometric sensor element for detecting fingerprints, the activation or deactivation is effected by touching and releasing the sensor element.

JP 2006-155455A discloses input device for performing collation of a fingerprint by an easy configuration of an electric circuit, and preventing the fingerprint collation from being performed by misoperation or the like to secure higher security.

JP 2009-032227A discloses a finger vein authentication apparatus applicable to a compact information processing apparatus like a mobile phone, and an information processing apparatus using the same. The finger vein authentication apparatus includes a light source for emitting light toward a finger, an image sensor for taking an image of the transmitted light from the finger, a lens apparatus for imaging the transmitted light to the image sensor, and an image processor for processing the taken image. The lens apparatus includes a short focus wide angle lens unit, and the image processor extracts a vein pattern of the finger upon correcting the strain of the taken image.

### SUMMARY

The present invention is defined by the appended claims.

### TECHNICAL PROBLEM

A problem to be solved by the present invention is to provide a biometric authentication device capable of saving power.

### SOLUTION TO PROBLEM

According to an embodiment, a biometric authentication device includes the features of claim 1.

### BRIEF DESCRIPTION OF THE DRAWINGS

FIG. 1 is a perspective view showing a configuration of a biometric authentication device according to a first embodiment.
FIG. 2 is a block diagram showing a configuration of the biometric authentication device.
FIG. 3 is an explanatory diagram schematically showing a configuration of the biometric authentication device.
FIG. 4 is a cross-sectional view schematically showing a configuration of an operation mechanism used in the biometric authentication device.
FIG. 5 is a perspective view showing an example of use of the biometric authentication device.
FIG. 6 is a cross-sectional view showing a configuration of an operation mechanism used in a biometric authentication device according to a second embodiment.
FIG. 7 is a cross-sectional view showing a configuration of an operation mechanism used in a biometric authentication device according to a third embodiment.
FIG. 8 is a cross-sectional view showing a configuration of an operation mechanism used in a biometric authentication device according to another embodiment.
FIG. 9 is a cross-sectional view showing a configuration of an operation mechanism used in a biometric authentication device according to another embodiment.

### DETAILED DESCRIPTION

### EMBODIMENTS

### (First Embodiment)

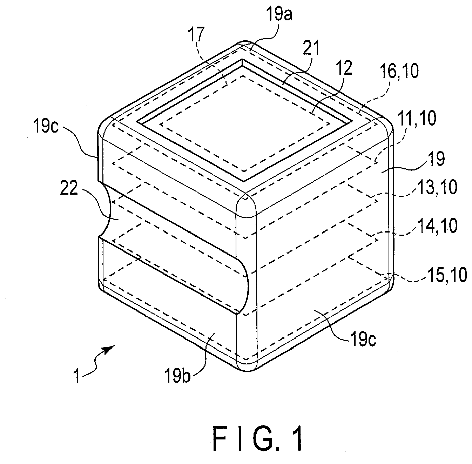

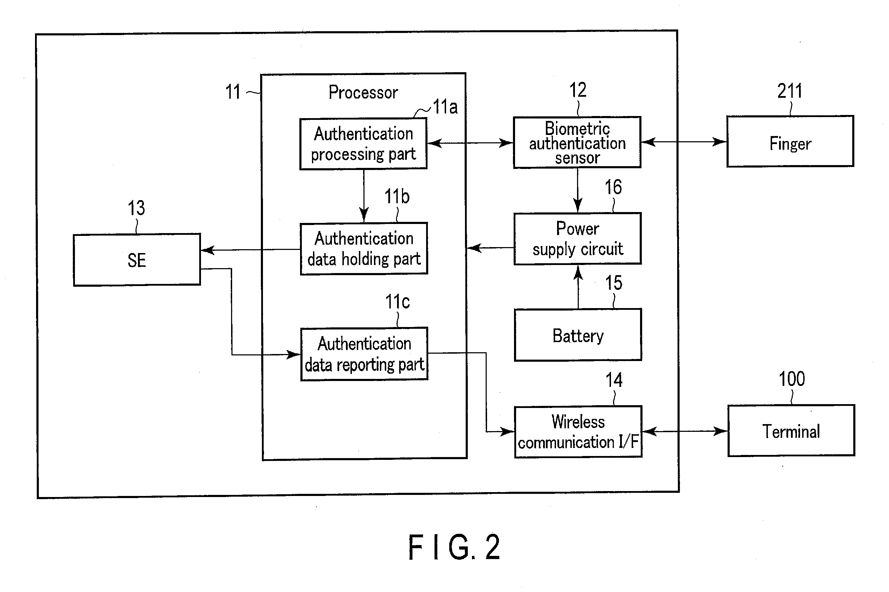

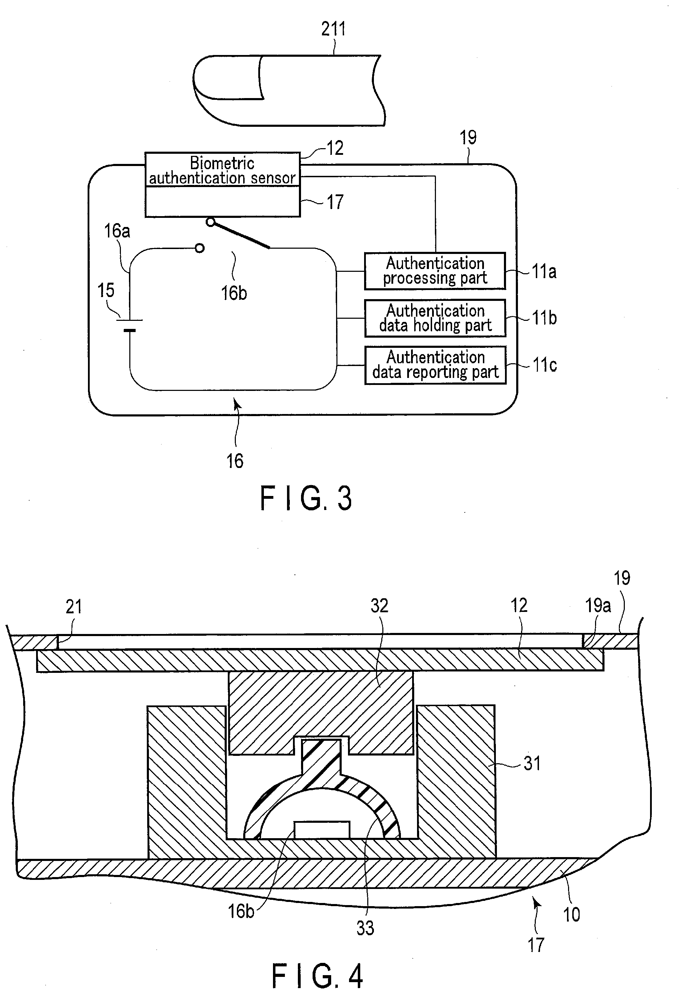

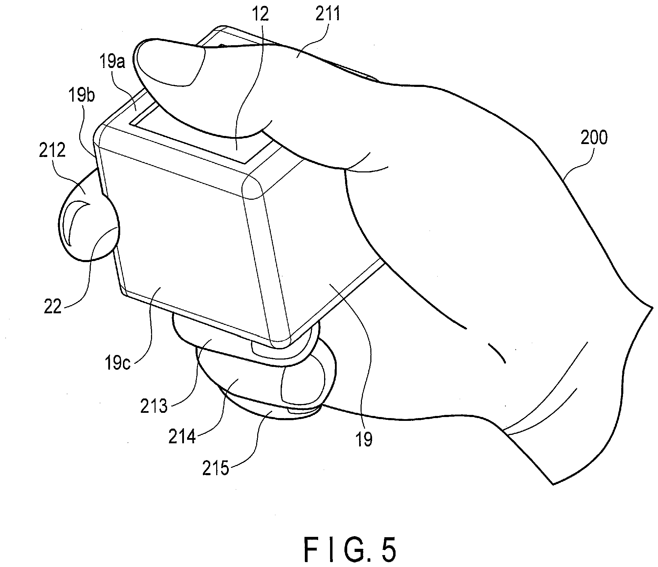

Hereinafter, a biometric authentication device 1 according to a first embodiment will be described with reference to FIGS. 1 to 4. FIG. 1 is a perspective view showing a configuration of the biometric authentication device 1 according to the first embodiment. FIG. 2 is a block diagram showing a configuration of the biometric authentication device 1. FIG. 3 is an explanatory diagram schematically showing a configuration of the biometric authentication device 1. FIG. 4 is a cross-sectional view schematically showing a configuration of an operation mechanism 17 used in the biometric authentication device 1. FIG. 5 is an explanatory diagram showing an example of use of the biometric authentication device 1. In each drawing, for convenience of description, the shape of each configuration is simplified or omitted, or the dimensions are enlarged or reduced.

As shown in FIGS. 1 to 3, the biometric authentication device 1 includes a processor 11, a biometric authentication sensor 12, a secure element (SE) 13 such as a central processing unit (CPU), a wireless communication interface (I/F) 14, a battery 15, a power supply circuit 16, and an operation mechanism 17. In addition, as shown in FIG. 1, the biometric authentication device 1 includes a housing 19 that houses the processor 11, the SE 13, the wireless communication I/F 14, the battery 15, the power supply circuit 16, and the operation mechanism 17 and exposes the biometric authentication sensor 12 to an outer surface.

For example, as shown in FIG. 1, the processor 11, the biometric authentication sensor 12, the SE 13, the wireless communication I/F 14, the battery 15, and the power supply circuit 16 used in the biometric authentication device 1 are mounted on a plurality of substrates 10. The plurality of substrates 10 are housed in the housing 19. The arrangement of the components shown in FIG. 1 is an example, and can be set as appropriate.

The processor 11 performs processing and control necessary for the operation of the biometric authentication device 1. The processor 11, for example, controls the biometric authentication sensor 12, the SE 13, and the wireless communication I/F 14 in order to realize various functions of the biometric authentication device 1 based on a program stored in the SE 13. As shown in FIGS. 2 and 3, the processor 11 functions as, for example, an authentication processing part 11a, an authentication data holding part 11b, and an authentication data reporting part 11c by executing a program stored in the SE 13.

The authentication processing part 11a, for example, encrypts, by the SE 13, biometric authentication information acquired by the biometric authentication sensor 12. The authentication data holding part 11b stores in the SE 13 the biometric authentication information encrypted by the authentication processing part 11a. The authentication data reporting part 11c reads out the encrypted biometric authentication information stored in the SE 13, and transmits the encrypted biometric authentication information via the wireless communication I/F 14 to a terminal 100 provided outside that performs biometric authentication.

The processor 11 is formed by one or more processing circuits. The processor 11 is, for example, a micro processing unit (MPU). Note that the processor 11 may be a central processing unit (CPU), a system on a chip (SoC), a digital signal processor (DSP), or a graphics processing unit (GPU). Alternatively, the processor 11 may be a combination thereof.

The biometric authentication sensor 12 is electrically connected to the processor 11. The biometric authentication sensor 12 is powered by the processor 11, for example. While power is supplied, the biometric authentication sensor 12 is controlled by, for example, the authentication processing part 11a of the processor 11, and acquires biometric authentication information of a finger 211 in contact with the biometric authentication sensor 12 as biometric authentication information of a living body. The biometric authentication sensor 12 transmits biometric authentication information acquired from the finger 211 to the authentication processing part 11a. The biometric authentication sensor 12 is, for example, a fingerprint sensor that acquires image data of a fingerprint from a finger. The biometric authentication sensor 12 is, for example, formed in a rectangular shape. The biometric authentication sensor 12 may be other than a fingerprint sensor, and may be, for example, a vein sensor that acquires a vein pattern of a finger as image data.

The SE 13 is electrically connected to the processor 11. The SE 13 is powered by the processor 11, for example. The SE 13 includes a memory capable of storing data and a cryptographic logic circuit. The SE 13 encrypts and stores the biometric authentication information. The SE 13 stores various programs for exhibiting a biometric authentication function. While power is supplied, the SE 13 is controlled by, for example, the authentication processing part 11a and the authentication data holding part 11b of the processor 11, and encrypts and stores the biometric authentication information. For example, the SE 13 holds stored information such as various programs even while power is not supplied.

The wireless communication I/F 14 is electrically connected to the processor 11. The wireless communication I/F 14 is, for example, powered by the processor 11. The wireless communication I/F 14 is an interface that transmits and receives information to and from the terminal 100 by wireless communication such as Bluetooth (registered trademark) or Wi-Fi (registered trademark). The battery 15 is, for example, a flat small battery. The battery 15 may be a primary battery or a secondary battery.

The power supply circuit 16 is a power source circuit. The power supply circuit 16 includes a circuit part 16a and a switch 16b provided in the circuit part 16a. In the power supply circuit 16, the circuit part 16a is normally open, and the circuit part 16a is closed when the switch 16b is operated. The circuit part 16a connects the processor 11 and the battery 15. In addition to the processor 11, the circuit part 16a may connect the biometric authentication sensor 12, the SE 13, and the wireless communication I/F 14 to the battery 15.

The circuit part 16a in the open state stops supply of current from the battery 15 to the processor 11. The circuit part 16a in the closed state supplies current from the battery 15 to the processor 11.

The switch 16b is normally in an OFF state, and is operated to enter an ON state. For example, the switch 16b closes the circuit part 16a while the switch 16b is operated and in the ON state. In other words, the switch 16b is, for example, a switch of a momentary system. For example, the switch 16b is mechanically or electrically operated by the operation mechanism 17.

In such a power supply circuit 16, the circuit part 16a is closed only while the switch 16b is operated, and power is supplied from the battery 15 to the processor 11. In other words, while the switch 16b is operated, the circuit part 16a is closed and power is supplied from the battery 15 to the processor 11, so that the authentication processing part 11a, the authentication data holding part 11b, and the authentication data reporting part 11c function. When the circuit part 16a is closed while the switch 16b is operated, power is also supplied to the biometric authentication sensor 12, the SE 13, and the wireless communication I/F 14 via the processor 11.

When biometric authentication is performed by the biometric authentication sensor 12, the operation mechanism 17 operates the switch 16b by bringing the finger 211 into contact with the biometric authentication sensor 12 and moving the biometric authentication sensor 12 in one direction with the finger 211.

The operation mechanism 17, for example, holds the biometric authentication sensor 12 in a movable manner. For example, the operation mechanism 17 is provided between the biometric authentication sensor 12 and the substrate 10 on which the biometric authentication sensor 12 is mounted. The operation mechanism 17, for example, holds the biometric authentication sensor 12 so as to be movable between an initial position of the biometric authentication sensor 12 and an operation position at which the biometric authentication sensor 12 moves to operate the switch 16b. Further, the operation mechanism 17 biases the biometric authentication sensor 12 to the initial position and positions the biometric authentication sensor 12 at the initial position when no external force is applied.

Here, the operation of the biometric authentication sensor 12 is, for example, an operation in which a finger is brought into close contact with the biometric authentication sensor 12 in order to acquire biometric authentication information in the biometric authentication sensor 12, and then a user further intentionally moves the biometric authentication sensor 12. Note that the operation of the biometric authentication sensor 12 may be an operation of the biometric authentication sensor 12 that is not intended by the user as long as the operation occurs in association with an action of acquiring biometric authentication information in the biometric authentication sensor 12. A specific example of an operation of the biometric authentication sensor 12 that is not intended by the user is a movement of the biometric authentication sensor 12 that is additionally caused by an action of the user bringing a finger into close contact with the biometric authentication sensor 12 in order to acquire biometric authentication information in the biometric authentication sensor 12 without knowing that the biometric authentication sensor 12 will move.

The movement of the biometric authentication sensor 12 may be any movement as long as the switch 16b can be operated, and may be a movement of the biometric authentication sensor 12 that can be recognized by the user or a movement of the biometric authentication sensor 12 that is difficult for the user to recognize. That is, an amount of the movement of the biometric authentication sensor 12 associated with the operation of the biometric authentication sensor 12 can be set as appropriate.

As a specific example, when the user brings the finger 211 into contact with the biometric authentication sensor 12 and further performs an operation of pressing the biometric authentication sensor 12 down with the finger 211 from this contact state, the operation mechanism 17 operates the switch 16b. That is, in the example of the present embodiment, a moving direction between the initial position and the operation position of the biometric authentication sensor 12 by the operation mechanism 17 is a direction along a pressing direction of the biometric authentication sensor 12.

Further, for example, when the operation of the biometric authentication sensor 12 is released, the operation mechanism 17 moves the biometric authentication sensor 12 to the initial position and releases the operation of the switch 16b. That is, the operation mechanism 17 is a mechanism of a momentary system that closes the switch 16b only while the biometric authentication sensor 12 is being operated.

As a specific example, the operation mechanism 17 is a mechanism of a membrane system as shown in FIG. 4. For example, the operation mechanism 17 includes a guiding portion 31 provided in the housing 19, a guided portion 32 that moves along the guiding portion 31, and a rubber dome 33 that is operated by the guided portion 32.

The guiding portion 31 is, for example, formed in a tubular shape. The guiding portion 31 is disposed around the switch 16b of the power supply circuit 16 provided on the substrate 10. For example, the biometric authentication sensor 12 is fixed to the guided portion 32. The guided portion 32 moves in one direction along the guiding portion 31.

The rubber dome 33 holds the biometric authentication sensor 12 fixed to the guided portion 32 at the initial position by biasing the guided portion 32. When the biometric authentication sensor 12 is pressed down and the guided portion 32 moves toward the inside of the housing 19 along the guiding portion 31, the rubber dome 33 is elastically deformed to operate the switch 16b. The switch 16b may adopt a configuration that provides a click sensation such as a "click" when operated. When the biometric authentication sensor 12 is released from being pressed down, the rubber dome 33 biases the guided portion 32 with a restoring force and moves the biometric authentication sensor 12 to the initial position.

The force required to elastically deform the rubber dome 33 and operate the switch 16b is set as appropriate. For example, the rubber dome 33 may be elastically deformed when the user further presses the biometric authentication sensor 12 down with the finger 211 after the finger 211 is brought into contact with the biometric authentication sensor 12. Further, for example, the rubber dome 33 may be elastically deformed by bringing the finger 211 into contact with the biometric authentication sensor 12 to such an extent that biometric authentication information can be acquired by the biometric authentication sensor 12. In the present embodiment, an example will be described in which the rubber dome 33 is elastically deformed when the user further presses the biometric authentication sensor 12 down by applying a predetermined force after bringing the finger into contact with the biometric authentication sensor 12.

The housing 19 is, for example, formed in a size that fits in fingers of one hand. Here, a size that fits in the fingers of one hand is, for example, a size that can be held by the fingers of one hand. If the configuration shown in FIG. 2 is adopted, the shape is irrelevant.

In the present embodiment, the housing 19 is formed into a cubic shape having a size that fits into a sphere having a diameter of 7 cm, specifically, a cubic shape having 4 cm sides or less. The housing 19 has, for example, a ridge portion and a corner portion formed in a curved surface shape.

As shown in FIG. 1, the housing 19, for example, accommodates the processor 11, the SE 13, the wireless communication I/F 14, and the battery 15 that are respectively mounted on the substrates 10. In addition, for example, the housing 19 accommodates the substrates 10 on which the biometric authentication sensor 12 and the power supply circuit 16 are mounted and the operation mechanism 17, and exposes the biometric authentication sensor 12 to a part of the outer surface. As a specific example, the housing 19 has, on one surface thereof, an opening 21 for exposing the biometric authentication sensor 12.

In addition, the housing 19 has a groove 22 in a part of the outer surface, which is at least a part of positions at which the fingers come into contact with the housing 19 when the finger 211 is brought into contact with the biometric authentication sensor 12. The groove 22 is, for example, provided at least at a position of the housing 19 that is touched by a finger among the fingers that is different from a finger that touches the biometric authentication sensor 12.

The groove 22 is, for example, formed to a depth at which a finger can contact a bottom surface of the groove 22. The bottom surface of the groove 22 is formed into a curved surface shape, for example. The shape of the groove 22 can be set as appropriate, and for example, the bottom surface may be formed into a planar shape. However, the groove 22 preferably has a depth and a shape such that when the housing 19 is held by the fingers, a pad of a finger comes into contact with the bottom surface of the groove 22.

In the present embodiment, as shown in FIG. 1, the groove 22 is provided on one surface 19b of four surfaces adjacent to a surface 19a having the opening 21 for exposing the biometric authentication sensor 12 of the housing 19. The groove 22 is provided on the center side of one surface 19b adjacent to the surface 19a having the opening 21 in a direction orthogonal to the surface 19a on which the opening 21 is provided. The groove 22 extends along a direction parallel to the surface 19a on which the opening 21 is provided. In addition, for example, the groove 22 is provided between two opposing surfaces 19c adjacent to both the surface 19b on which the groove 22 is provided and the surface 19a having the opening 21 of the housing 19.

Next, an example of biometric authentication using the biometric authentication device 1 with the above-described configuration will be described. In the present embodiment, the finger 211 that performs biometric authentication using the biometric authentication device 1 will be described below as a thumb 211 of a right hand 200.

First, in the biometric authentication device 1 in which the finger 211 is not in contact with the biometric authentication sensor 12 and biometric authentication is not performed, the switch 16b is in the OFF state and the power supply circuit 16 is open. Thus, in the biometric authentication device 1, the supply of power from the battery 15 to the processor 11 is stopped. Therefore, the functions of the authentication processing part 11a, the authentication data holding part 11b, and the authentication data reporting part 11c of the processor 11, as well as the biometric authentication sensor 12, the SE 13, and the wireless communication I/F 14 are stopped.

When fingerprint authentication is performed, the user holds the housing 19 with either a left or right hand; in the present embodiment, the right hand 200. Specifically, for example, as shown in FIG. 5, the user places an index finger 212 in the groove 22 and supports a surface of the housing 19 opposing the surface 19a where the opening 21 is provided with a middle finger 213, a ring finger 214, and a little finger 215. Then, the user brings the thumb 211 into contact with the biometric authentication sensor 12.

When the user brings the thumb 211 into contact with the biometric authentication sensor 12 and presses the biometric authentication sensor 12 down with the thumb 211 so that the thumb 211 further closely contacts the biometric authentication sensor 12, the biometric authentication sensor 12 is moved by the operation mechanism 17. Then, the switch 16b is operated by the operation mechanism 17, and power is supplied from the battery 15 to the processor 11 by the power supply circuit 16.

More specifically, for example, when the biometric authentication sensor 12 is pressed down by the thumb 211 of the user, the guided portion 32 moves along the guiding portion 31 and elastically deforms the rubber dome 33. Then, the switch 16b is operated by the elastically deformed rubber dome 33. As a result, the switch 16b is turned on and the circuit part 16a is closed, so that supply of power from the battery 15 to the processor 11 is started.

When the supply of power from the battery 15 to the processor 11 is started, the processor 11 executes the biometric authentication function by the functions of the authentication processing part 11a, the authentication data holding part 11b, and the authentication data reporting part 11c.

Specifically, the authentication processing part 11a activates the biometric authentication sensor 12, and acquires biometric authentication information of a contact region of the thumb 211. In the present embodiment, since the biometric authentication sensor 12 is a fingerprint sensor, the authentication processing part 11a acquires fingerprint data of the thumb 211 in contact with the fingerprint sensor as biometric authentication information. Then, for example, the authentication processing part 11a encrypts by the SE 13 the biometric authentication information acquired by the biometric authentication sensor 12.

Next, the authentication data holding part 11b stores in the SE 13 the biometric authentication information encrypted by the authentication processing part 11a. Then, the authentication data reporting part 11c reads out the encrypted biometric authentication information stored in the SE 13, and transmits the biometric authentication information to the terminal 100 that is provided outside and performs biometric authentication via the wireless communication I/F 14.

As described above, while the biometric authentication sensor 12 is pressed down by the finger 211, the processor 11 acquires biometric authentication information, encrypts the biometric authentication information, stores the encrypted biometric authentication information, and transmits the stored encrypted biometric authentication information to the external terminal 100. Note that processes of encrypting, storing, and transmitting biometric authentication information are not limited to the above, and various changes can be made as necessary.

When the encrypted biometric authentication information is transmitted to the external terminal 100, the user releases the thumb 211 away from the biometric authentication sensor 12. When the thumb 211 separates from the biometric authentication sensor 12, the guided portion 32 is biased by restoration of the rubber dome 33, and the biometric authentication sensor 12 returns to the initial position. As a result, the operation of the switch 16b is released, the switch 16b is turned off, and the circuit part 16a is opened. Thus, the supply of power from the battery 15 to the processor 11 is stopped.

As described above, while the user presses the biometric authentication sensor 12 down with a finger such as the thumb 211, power is supplied from the battery 15 to the processor 11 by the power supply circuit 16, and the biometric authentication function is executed by the processor 11.

When the biometric authentication sensor 12 is operated in a state in which any finger is in contact with the biometric authentication sensor 12 on a next occasion, power is supplied to the processor 11 and the biometric authentication function is executed while the biometric authentication sensor 12 is operated (pressed down) in the same manner.

According to the biometric authentication device 1 with the above-described configuration, the power supply and the execution of the biometric authentication function can be continuously performed by bringing the finger 211 into contact with the biometric authentication sensor 12 or by bringing the finger 211 into contact with the biometric authentication sensor 12 and then further operating the biometric authentication sensor 12 with the finger 211 in contact.

That is, the biometric authentication device 1 closes the power supply circuit 16 by bringing the finger 211 into contact with the biometric authentication sensor 12 and operating the switch 16b in order to acquire biometric authentication information. Then, in the biometric authentication device 1, power is supplied to the processor 11 only when the power supply circuit 16 is closed, and a fingerprint authentication function of acquisition, encryption, storage, and communication of biometric authentication information is exhibited. Therefore, the biometric authentication device 1 can supply electric power from the battery 15 to the processor 11 only when a finger is in contact with the biometric authentication sensor 12 in order to exhibit the fingerprint authentication function. Since power is not supplied from the battery 15 to the processor 11 during standby, the biometric authentication device 1 can save power.

An instruction to supply power to the processor 11 may be an operation in which the user brings the finger 211 into contact with the biometric authentication sensor 12 in order to acquire biometric authentication information or an operation in which the user brings the finger 211 into contact with the biometric authentication sensor 12 and moves the finger 211 in the contact state to operate the biometric authentication sensor 12. Thus, the biometric authentication device 1 does not require the user to be aware of power supply, such as operating a power switch, and is user-friendly, and thus has high usability. In addition, the biometric authentication device 1 can cause the user to consciously perform the operation of pressing the biometric authentication sensor 12 down for power supply when acquiring biometric authentication information. That is, when the user recognizes the pressing down of the biometric authentication sensor 12 and presses the biometric authentication sensor 12, the finger 211 comes into close contact with the biometric authentication sensor 12. Therefore, an acquisition efficiency of the biometric authentication information can be improved.

Further, for example, the movement amount of the biometric authentication sensor 12 and a force required to move the biometric authentication sensor 12 can be adjusted as appropriate by the configuration of the operation mechanism 17. Therefore, a response of the biometric authentication device 1 to an operation of the user can be appropriately set by the configuration of the operation mechanism 17.

That is, in order to give the user a feeling of starting biometric authentication or a feeling of performing biometric authentication, the movement amount of the biometric authentication sensor 12 by the operation mechanism 17 and the force required for the movement may be set to be relatively large. In addition, if it is not desired to make the user aware of a feeling of starting biometric authentication, a feeling of performing biometric authentication, or an operation feeling of the biometric authentication sensor 12, for example, the operation mechanism 17 may be set to have a small movement amount of the biometric authentication sensor 12 and a small force required for the movement.

In the present embodiment, an example has been described in which the rubber dome 33 is elastically deformed when the user presses the biometric authentication sensor 12 down by further applying a predetermined force after bringing a finger into contact with the biometric authentication sensor 12. Such an operation mechanism 17 brings the finger 211 into close contact with the biometric authentication sensor 12, thereby improving the acquisition efficiency of biometric authentication information and allowing the user to realize that biometric authentication is being performed.

As described above, the biometric authentication device 1 according to the present embodiment is configured such that the power supply circuit 16 is closed when a finger is placed on the biometric authentication sensor 12. As a result, the biometric authentication device 1 supplies power to the processor 11 only when the biometric authentication function is exhibited, and thus power can be saved.

### (Second Embodiment)

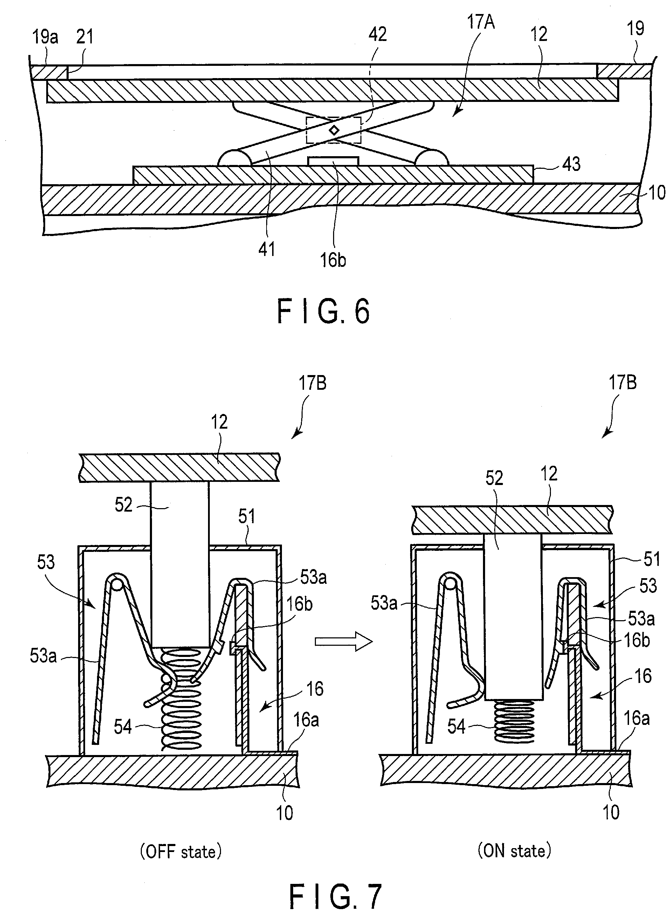

Hereinafter, a biometric authentication device 1 according to a second embodiment will be described with reference to FIG. 6. FIG. 6 is a cross-sectional view schematically showing a configuration of an operation mechanism 17A used in the biometric authentication device 1 according to the second embodiment. In the biometric authentication device 1 according to the second embodiment, the configuration of the operation mechanism 17A is different from the configuration of the biometric authentication device 1 according to the first embodiment described above, and other similar configurations are denoted by the same reference numerals and detailed descriptions thereof are omitted.

The operation mechanism 17A used in the biometric authentication device 1 holds the biometric authentication sensor 12 in a movable manner. For example, the operation mechanism 17A holds the biometric authentication sensor 12 so as to be movable between an initial position and an operation position where the switch 16b is operated. Further, for example, the operation mechanism 17A biases the biometric authentication sensor 12 to the initial position, and positions the biometric authentication sensor 12 at the initial position when no external forces are applied. As a specific example, when the finger 211 comes into contact with the biometric authentication sensor 12 and the biometric authentication sensor 12 is further operated by the finger 211 in one direction, the operation mechanism 17A moves the biometric authentication sensor 12 from the initial position to the operation position and operates the switch 16b.

A moving direction of the biometric authentication sensor 12 between the initial position and the operation position is the pressing direction thereof, and the operation mechanism 17A moves the biometric authentication sensor 12 into the housing 19 when the biometric authentication sensor 12 is pressed down by the finger 211 from a state in which the finger 211 is in contact with the biometric authentication sensor 12. Further, for example, when the operation of the biometric authentication sensor 12 is released, the operation mechanism 17A moves the biometric authentication sensor 12 to the initial position. That is, the operation mechanism 17A is a mechanism of a momentary system that closes the switch 16b only while the biometric authentication sensor 12 is being operated.

As a specific example, as shown in FIG. 6, the operation mechanism 17A is a mechanism of a pantograph system. For example, the operation mechanism 17A includes an arm 41 provided in the housing 19, a biasing member 42 that biases the arm 41 toward the initial position, and a base 43 that fixes the arm 41 to the substrate 10. The arm 41 is disposed above the switch 16b of the power supply circuit 16 provided on the substrate 10. The arm 41 holds the biometric authentication sensor 12 at the initial position, for example. The arm 41 is operated by pressing the biometric authentication sensor 12 down. When the arm 41 is operated, the height thereof is reduced and the switch 16b is operated. When the pressing down of the biometric authentication sensor 12 is released, the height of the arm 41 returns due to the biasing by the biasing member 42, and the arm 41 moves the biometric authentication sensor 12 to the initial position.

The biasing member 42 is an elastic body such as a spring. The biasing member 42 biases the arm 41 so that the arm 41 has a predetermined height when the pressing down of the biometric authentication sensor 12 is released.

An amount of movement of the arm 41 and a force required to operate the arm 41 against the biasing of the biasing member 42 so as to operate the switch 16b are appropriately set depending on the dimensions and shape of the arm 41 and the configuration of the biasing member 42.

For example, such a switch 16b or operation mechanism 17A may have a configuration that provides a click sensation.

As described above, the biometric authentication device 1 using the operation mechanism 17A according to the second embodiment is configured to close the power supply circuit 16 by bringing a finger into contact with the biometric authentication sensor 12 in the same manner as the biometric authentication device 1 according to the first embodiment described above. Thus, in the biometric authentication device 1, power is supplied to the processor 11 only when the biometric authentication function is exhibited. Therefore, the biometric authentication device 1 can save power.

### (Third Embodiment)

Hereinafter, a biometric authentication device 1 according to a third embodiment will be described with reference to FIG. 7. FIG. 7 is a cross-sectional view schematically showing a configuration of an operation mechanism 17B used in the biometric authentication device 1 according to the third embodiment. In FIG. 7, a left diagram shows an (OFF state) before the switch 16b is operated by the operation mechanism 17B, and a right diagram shows an (ON state) after the switch 16b is operated by the operation mechanism 17B. In the biometric authentication device 1 according to the third embodiment, the configuration of the operation mechanism 17B is different from the configuration of the biometric authentication device 1 according to the first embodiment described above, and other similar configurations are denoted by the same reference numerals and detailed descriptions thereof are omitted.

The operation mechanism 17B used in the biometric authentication device 1 holds the biometric authentication sensor 12 in a movable manner. For example, the operation mechanism 17B holds the biometric authentication sensor 12 so as to be movable between an initial position and an operation position where the switch 16b is operated. Further, for example, the operation mechanism 17B biases the biometric authentication sensor 12 to the initial position, and positions the biometric authentication sensor 12 at the initial position when no external forces are applied. As a specific example, when the finger 211 comes into contact with the biometric authentication sensor 12 and the biometric authentication sensor 12 is further operated by the finger 211 in one direction, the operation mechanism 17B moves the biometric authentication sensor 12 from the initial position to the operation position and operates the switch 16b.

A moving direction of the biometric authentication sensor 12 between the initial position and the operation position is the pressing direction thereof, and the operation mechanism 17B moves the biometric authentication sensor 12 into the housing 19 when the biometric authentication sensor 12 is pressed down by the finger 211 from a state in which the finger 211 is in contact with the biometric authentication sensor 12. Further, for example, when the operation of the biometric authentication sensor 12 is released, the operation mechanism 17B moves the biometric authentication sensor 12 to the initial position. That is, the operation mechanism 17B is a mechanism of a momentary system that closes the switch 16b only while the biometric authentication sensor 12 is being operated.

In addition, for example, when the biometric authentication sensor 12 is operated and the switch 16b is switched from the OFF state to the ON state and from the ON state to the OFF state, the operation mechanism 17B generates a click sensation and a typing sound.

As a specific example, the operation mechanism 17B is a mechanism of a mechanical system as shown in FIG. 7. For example, the operation mechanism 17B includes a guiding portion 51 provided in the housing 19, a guided portion 52 that moves along the guiding portion 51, an operation portion 53 that moves in a direction intersecting a moving direction of the guided portion 52 by being pressed by the guided portion 52, and a coil spring 54 that biases the guided portion 52.

The guiding portion 51 is formed in a tubular shape, and holds the guided portion 52 so as to be movable in one direction. For example, the biometric authentication sensor 12 is fixed to the guided portion 52. The guided portion 52 moves along the guiding portion 51.

The operation portion 53 is formed of, for example, a metallic material, and includes a pair of members 53a that are moved by the guided portion 52 in a direction intersecting the moving direction of the guided portion 52 when the guided portion 52 moves along the guiding portion 51.

One member 53a of the operation portion 53 is disposed to face the switch 16b of the power supply circuit 16 and has a contact point with the switch 16b. When the guided portion 52 moves in one direction from the (OFF state) to the (ON state) in FIG. 7, the operation portion 53 moves in a direction in which the pair of members 53a provided with the switch 16b are separated. Then, when one member 53a facing the switch 16b moves toward the switch 16b and the one member 53a comes into contact with the switch 16b, the switch 16b is turned on. As described above, when the switch 16b is operated by the operation portion 53, the power supply circuit 16 is closed.

In addition, the operation portion 53 is operated by the guided portion 52, and generates a click sensation or a typing sound when one member 53a comes into contact with the switch 16b and when the switch 16b is separated from the contact state.

As described above, the biometric authentication device 1 using the operation mechanism 17B according to the third embodiment is configured to close the power supply circuit 16 by bringing a finger into contact with the biometric authentication sensor 12 in the same manner as the biometric authentication device 1 according to the first embodiment described above. Thus, in the biometric authentication device 1, power is supplied to the processor 11 only when the biometric authentication function is exhibited. Therefore, the biometric authentication device 1 can save power. In addition, since the operation mechanism 17B is of a mechanical system capable of generating a click sensation and a typing sound, it is possible to preferably transmit a feeling that biometric authentication has started and a feeling that biometric authentication has ended to a user.

The above-described embodiments are presented by way of example only, and the biometric authentication device is not limited to the above-described embodiments. For example, in the above-described examples, examples having mechanisms of a membrane system, a pantograph system, and a mechanical system as the operation mechanisms 17, 17A, and 17B, respectively, have been described, but the present invention is not limited thereto.

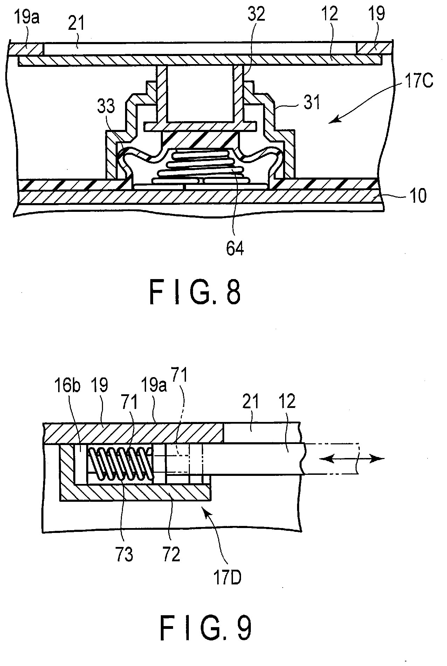

For example, as shown in another embodiment of FIG. 8, an operation mechanism 17C may be a mechanism of a non-contact electrostatic capacity system. Such an operation mechanism 17C includes, for example, the rubber dome 33 and a spring 64 such as a conical spring compressed by the rubber dome 33 below the guided portion 32 moving along the guiding portion 31. Then, the operation mechanism 17C detects an electrostatic capacity that changes due to deformation of the spring 64, recognizes movement of the biometric authentication sensor 12 provided in the guided portion 32, and turns on the switch 16b.

Such an operation mechanism 17C can obtain the same effects as those of the operation mechanisms 17, 17A, and 17B described above. Further, by applying the mechanism of the non-contact electrostatic capacity system to the operation mechanism 17C, a contact for turning on/off the switch 16b is not required, so that the operation mechanism 17C has a high durability. In addition, since generation of a click sensation or sound can be suppressed, the operation mechanism 17C of the non-contact electrostatic capacity system is suitable for a case where generation of a click sensation or sound is desired to be suppressed during use of the biometric authentication device 1. In a case where a click sensation is desired even when the operation mechanism 17C of the non-contact electrostatic capacity system is used, for example, a configuration that provides a click sensation may be adopted for the switch 16b.

Further, as shown in another embodiment of FIG. 9, an operation mechanism 17D may turn on the switch 16b by slidably moving the biometric authentication sensor 12 in one direction of a main surface direction of the biometric authentication sensor 12 as an operation direction from a user. For example, such an operation mechanism 17D includes an operation body 71 that is provided in the biometric authentication sensor 12 and operates the switch 16b, a guiding portion 72 that guides the sliding movement of the operation body 71 along the main surface direction of the biometric authentication sensor 12, and a biasing member 73 that biases the biometric authentication sensor 12 toward an initial position.

In such an operation mechanism 17D, after a finger is brought into close contact with the biometric authentication sensor 12, the finger 211 is moved along a surface direction of the biometric authentication sensor 12, whereby the biometric authentication sensor 12 is slidably moved and the operation body 71 is moved. When the operation body 71 moves from a position indicated by a two-dot chain line to a position indicated by a solid line in FIG. 9, for example, a tip of the operation body 71 operates the switch 16b. As a result, the operation mechanism 17D can turn on the switch 16b and close the power supply circuit 16. Thus, such an operation mechanism 17D can obtain the same effects as those of the above-described operation mechanisms 17, 17A, 17B, and 17C.

In addition, since the operation mechanism 17D presses the finger 211 against the biometric authentication sensor 12 and moves the biometric authentication sensor 12, a state in which the finger 211 is in close contact with the biometric authentication sensor 12 is maintained when moving the biometric authentication sensor 12. Therefore, the biometric authentication device 1 including the operation mechanism 17D can improve an acquisition efficiency of biometric authentication information.

Further, as is clear from the operation mechanism 17D, the moving direction of the biometric authentication sensor 12 for the operation of the switch 16b by the operation mechanism in the present embodiment is not limited to the pressing direction. That is, as long as the biometric authentication sensor 12 can be moved in a state in which the finger 211 is in contact with the biometric authentication sensor 12, a direction other than that of the above-described operation mechanisms can be appropriately set.

Further, for example, in each of the operation mechanisms 17 described above, an example of a momentary system that closes the switch 16b only while the biometric authentication sensor 12 is being operated has been described as an operation system, but the operation system is not limited thereto. For example, the operation mechanism 17 may have a configuration of an alternate system in which once the biometric authentication sensor 12 is operated, the switch 16b is turned on, the on state of the switch 16b is maintained even if the operation of the biometric authentication sensor 12 is released, and the switch 16b is turned off by operating the biometric authentication sensor 12 again.

Further, for example, the operation mechanism 17 may not be of an alternate system, and the switch 16b may be of an alternate type. That is, the switch 16b may be turned on when operated once, and may be turned off when operated next.

An example of the switch 16b and operation mechanism 17 of an alternate system is a toggle switch, but other configurations may be employed. For example, the operation mechanism 17 may be a combination of a mechanism of a membrane, pantograph, or mechanical system and a mechanism of an alternate system in which a movement in a returning direction is restricted when pressed down and the restriction on the movement in the returning direction is released when pressed down next to move the biometric authentication sensor 12 to the initial position.

When the switch 16b or the operation mechanism 17 of the alternate system is used, the biometric authentication device 1 may include a switching portion that forcibly switches the switch 16b from the ON state to the OFF state after a predetermined time has elapsed. For example, since the switching portion can prevent power from being continuously supplied to the processor 11 due to forgetting to switch the switch 16b to the OFF state after switching the switch 16b to the ON state, it is effective for power saving. The switching portion may have a configuration in which the switch 16b is electrically switched or mechanically switched as long as the switch 16b can be switched to the OFF state after a predetermined time has elapsed.

Further, the biometric authentication device 1 described above has a configuration in which the biometric authentication sensor 12 is operated by bringing a finger into contact with the sensor. Therefore, in order to prevent an erroneous operation in which the switch 16b is operated by an object, etc. other than the finger 211 coming into contact with the biometric authentication sensor 12, the biometric authentication device 1 may further include a lock mechanism that locks the operation of the biometric authentication sensor 12 or a cover that covers the biometric authentication sensor 12. With such a configuration, it is possible to prevent the power supply circuit 16 from being closed and power from being supplied from the battery 15 to the processor 11 when dirt adhering to the biometric authentication sensor 12 is cleaned off or due to an erroneous operation, etc.

In addition, in the above-described examples, the configuration of the housing 19 in which the groove 22 is provided on one outer surface has been described, but the configuration is not limited thereto. For example, a configuration in which grooves are provided on a pair of outer surfaces facing each other may be employed, or a configuration in which grooves are provided on three surfaces may be employed. Further, the shape of the groove 22 is not limited to a shape extending in one direction, and may be, for example, a shape following the shape of a finger supporting the housing 19. Furthermore, the housing 19 may be formed without a groove. However, in a case where the same user performs biometric authentication, in order to bring the same finger into contact with the biometric authentication sensor 12 in the same posture, a configuration in which a groove, etc. is provided as a guiding portion that guides the position of the fingers in any part of the housing 19 is preferable. In addition, the housing 19 is not limited to a cubic shape as long as it has a size that fits in the fingers of one hand, and may be, for example, a rectangular parallelepiped shape or a spherical shape.

In the above-described examples, the biometric authentication device 1 has the configuration in which power is supplied to the processor 11 to exhibit the biometric authentication function by touching the biometric authentication sensor 12 with a finger or further operating the biometric authentication sensor 12 after the touch. However, the biometric authentication device 1 may include a notification device that notifies the outside of execution of a predetermined process such as power supply to the processor 11 or exhibition of a biometric authentication function. Such a biometric authentication device 1 includes, for example, a light source such as an LED that emits light and/or a sound source such as a speaker that emits sound as the notification device. Then, in the biometric authentication device 1, the processor 11 controls the notification device to notify the outside of execution of each process by light or sound. The biometric authentication device 1 may have a configuration such that it can be determined which process is being executed by changing a pattern of light or sound.

In the above-described examples, the biometric authentication device 1 has the configuration in which power is supplied to the processor 11 to exhibit a biometric authentication function by touching the biometric authentication sensor 12 with a finger or further operating the biometric authentication sensor 12 after the touch. However, the biometric authentication device 1 may have a configuration in which a biometric authentication function is exhibited by operating the biometric authentication sensor 12 twice.

Specifically, for example, when a finger is pressed against the biometric authentication sensor 12 to perform the first operation of the biometric authentication sensor 12, the switch 16b is operated by the operation of the biometric authentication sensor 12, the circuit part 16a of the power supply circuit 16, which is a power source circuit, is closed, and the processor 11 and the battery 15 are connected in the biometric authentication device 1. As a result, power is supplied to the processor 11.

Then, once the finger is released from the biometric authentication sensor 12, the finger is pressed against the biometric authentication sensor 12 again to perform the second operation of the biometric authentication sensor 12, and the switch 16b is operated by the movement of the biometric authentication sensor 12. When the processor 11 to which power is supplied detects this operation of the switch 16b, the processor 11 executes a program stored in the SE 13 and executes functions as the authentication processing part 11a, the authentication data holding part 11b, and the authentication data reporting part 11c. Then, the processor 11 performs biometric authentication processing such as fingerprint authentication of the finger pressed against the biometric authentication sensor 12, and transmits biometric authentication information to the terminal 100. As described above, the biometric authentication device 1 may have a configuration in which power is supplied to the processor 11 by the first operation of the biometric authentication sensor 12, and the processor 11 performs biometric authentication processing by the second operation of the biometric authentication sensor 12.

When power is supplied to the processor 11 by the first operation of the biometric authentication sensor 12, the biometric authentication device 1 may notify the outside of information indicating that power has been supplied to the processor 11 by sound or light using the notification device. Similarly, when the biometric authentication function is being executed by the processor 11 by the second operation of the biometric authentication sensor 12, information on the execution of the biometric authentication function may be notified to the outside by sound or light using the notification device.

Although the biometric authentication device 1 of the above-described examples has a configuration in which power is supplied to the processor 11 by the first operation of the biometric authentication sensor 12 and the processor 11 executes a program by the second operation of the biometric authentication sensor 12, the configuration is not limited thereto. For example, by the first operation of the biometric authentication sensor 12, a part of the circuit part 16a of the power supply circuit 16, which is a power source circuit, may be closed, the processor 11 and the battery 15 may be connected, and power may be supplied to the processor 11. Then, by the second operation of the biometric authentication sensor 12, a part of the circuit part 16a of the power supply circuit 16, which is a power source circuit, may be closed, and the biometric authentication sensor 12, the SE 13, and the wireless communication I/F 14 may be connected to the battery 15, for example, so as to shift to a state where biometric authentication can be performed.

In these examples, the biometric authentication device 1 has a configuration in which power is supplied to the processor 11 and a biometric authentication function is executed when the biometric authentication sensor 12 is operated twice, but the configuration is not limited thereto.

For example, a configuration in which when the biometric authentication sensor 12 is pressed with a finger, the movement of the biometric authentication sensor 12 is detectable by the switch 16b, a sensor, etc. at two portions in the moving direction of the biometric authentication sensor 12 may be employed. That is, the biometric authentication device 1 may sequentially perform the power supply to the processor 11 and the execution of the biometric authentication function with different pressing amounts of the biometric authentication sensor 12 in one operation of the biometric authentication sensor 12.

As a specific example, in the biometric authentication device 1, after the biometric authentication sensor 12 is touched with a finger, the biometric authentication sensor 12 is operated by an operation of the biometric authentication sensor 12 such as pressing down, whereby the movement amount of the biometric authentication sensor 12 increases. When the movement amount reaches a predetermined movement amount, first, the circuit part 16a of the power supply circuit 16, which is a power source circuit, is closed, and the processor 11 and the battery 15 are connected. As a result, power is supplied to the processor 11.

When the operation of the biometric authentication sensor 12 is further continued, the movement amount of the biometric authentication sensor 12 further increases, and when the predetermined movement amount is reached, the switch 16b is operated by the moved biometric authentication sensor 12. Then, when the processor 11 detects the operation of the switch 16b, the processor 11 executes a program stored in the SE 13 and executes functions as the authentication processing part 11a, the authentication data holding part 11b, and the authentication data reporting part 11c.

As described above, the biometric authentication device 1 may have a configuration in which the power supply to the processor 11 and the execution of the biometric authentication function are sequentially performed according to the movement amount of the biometric authentication sensor 12. In the case of such a biometric authentication device 1, a configuration in which the operation mechanism 17 generates a click sensation in a finger pressing the biometric authentication sensor 12 each time the movement amount of the biometric authentication sensor 12 reaches a predetermined movement amount may be employed.

The above-described biometric authentication device 1 supplies power to the processor 11 and exhibits a fingerprint authentication function by the biometric authentication sensor 12 being touched with a finger or the biometric authentication sensor 12 being further operated after the touch. Thus, for example, the biometric authentication device 1 may include a notification means that notifies the start and end of the biometric authentication function by light, sound, etc. However, from the viewpoint of power saving, it is preferable to use a configuration with low power consumption for the notification means.

According to the biometric authentication device of at least one embodiment described above, power saving can be achieved by supplying power to the processor only when the biometric authentication function is exhibited.

## Claims

1. A biometric authentication device (1) comprising:
a biometric authentication sensor (12) configured to be brought into contact with any finger of a hand and acquire biometric authentication information from the finger;
a housing (19) accommodating the biometric authentication sensor (12), including an opening (21) exposing the biometric authentication sensor (12) to a part of an outer surface, and configured to be held by the fingers of the hand, the housing (19) having a cubic shape that can be fitted into a sphere having a diameter of 7 cm;
a battery (15) provided in the housing (19); and
a power supply circuit (16) configured to start supply of power from the battery (15) by the biometric authentication sensor (12) being pressed down in a state where the finger is in contact with the biometric authentication sensor (12);
wherein a groove (22) is provided in the housing (19), the groove 22 being provided in one surface (19b) of the four surfaces adjacent to a surface (19a) with the opening (21) where the biometric authentication sensor (12) is exposed.

2. The biometric authentication device (1) according to claim 1, wherein
the power supply circuit (16) includes a circuit part (16a) configured to connect the battery (15) and the biometric authentication sensor (12), and a switch configured to open the circuit part (16a) and close the circuit part (16a) when operated, and
the biometric authentication device (1) further comprises an operation mechanism (17) configured to operate the switch by the biometric authentication sensor (12) being pressed down in a state where the finger is in contact with the biometric authentication sensor (12).

3. The biometric authentication device (1) according to claim 2, comprising:
an authentication processing part (11a) connected to the circuit part (16a), and configured to authenticate the biometric authentication information based on information acquired by the biometric authentication sensor (12) when the circuit part (16a) is closed; and
an authentication data holding part (11b) connected to the circuit part (16a), and configured to store the biometric authentication information processed by the authentication processing part (11a) when the circuit part (16a) is closed.

4. The biometric authentication device (1) according to claim 3, comprising:
a communication interface configured to communicate with an external terminal; and
an authentication data reporting part connected to the circuit part (16a), and configured to transmit the biometric authentication information stored in the authentication data holding part (11b) to the terminal via the communication interface when the circuit part (16a) is closed.

5. The biometric authentication device (1) according to any one of claims 2 to 4, wherein the operation mechanism (17) holds the biometric authentication sensor (12) to be movable between an initial position and a position at which the switch is operated, and biases the biometric authentication sensor (12) toward the initial position.

6. The biometric authentication device (1) according to claim 5, wherein the operation mechanism (17) and the switch are of a momentary system.

7. The biometric authentication device (1) according to claim 5, wherein the operation mechanism (17) or the switch is of an alternate system.

8. The biometric authentication device (1) according to any one of claims 1 to 7, wherein the biometric authentication sensor (12) is a fingerprint sensor.

## Patentansprüche

1. Biometrische Authentifizierungsvorrichtung (1), aufweisend:
einen biometrischen Authentifizierungssensor (12), der eingerichtet ist, mit einem beliebigen Finger einer Hand in Kontakt gebracht zu werden und biometrische Authentifizierungsinformationen von dem Finger zu erfassen;
ein Gehäuse (19), das den biometrischen Authentifizierungssensor (12) aufnimmt, mit einer Öffnung (21), die den biometrischen Authentifizierungssensor (12) zu einem Teil einer Außenfläche freilegt, und eingerichtet ist, von den Fingern einer Hand gehalten zu werden, wobei das Gehäuse (19) eine kubische Form aufweist, die in eine Kugel mit einem Durchmesser von 7 cm eingepasst werden kann;
eine Batterie (15), die in dem Gehäuse (19) vorgesehen ist; und
eine Stromversorgungsschaltung (16), die eingerichtet ist, die Zufuhr von Strom von der Batterie (15) zu beginnen, indem der biometrische Authentifizierungssensor (12) in einem Zustand niedergedrückt wird, in dem der Finger mit dem biometrischen Authentifizierungssensor (12) in Kontakt ist;
wobei eine Nut (22) in dem Gehäuse (19) vorgesehen ist, wobei die Nut (22) in einer Fläche (19b) der vier Flächen benachbart zu einer Fläche (19a) mit der Öffnung (21) vorgesehen ist, wo der biometrische Authentifizierungssensor (12) freiliegt.

2. Biometrische Authentifizierungsvorrichtung (1) nach Anspruch 1, wobei
die Stromversorgungsschaltung (16) einen Schaltungsteil (16a), der eingerichtet ist, die Batterie (15) und den biometrischen Authentifizierungssensor (12) zu verbinden, und einen Schalter aufweist, der eingerichtet ist, den Schaltungsteil (16a) zu öffnen und den Schaltungsteil (16a) zu schließen, wenn er betätigt wird, und
die biometrische Authentifizierungsvorrichtung (1) ferner einen Betätigungsmechanismus (17) aufweist, der eingerichtet ist, den Schalter zu betätigen, indem der biometrische Authentifizierungssensor (12) in einem Zustand niedergedrückt wird, in dem der Finger mit dem biometrischen Authentifizierungssensor (12) in Kontakt ist.

3. Biometrische Authentifizierungsvorrichtung (1) nach Anspruch 2, aufweisend:
einen Authentifizierungsverarbeitungsteil (11a), der mit dem Schaltungsteil (16a) verbunden ist und eingerichtet ist, die biometrischen Authentifizierungsinformationen basierend auf Informationen zu authentifizieren, die durch den biometrischen Authentifizierungssensor (12) erfasst werden, wenn der Schaltungsteil (16a) geschlossen ist; und
einen Authentifizierungsdatenhalteteil (11b), der mit dem Schaltungsteil (16a) verbunden ist und eingerichtet ist, die biometrischen Authentifizierungsinformationen zu speichern, die durch den Authentifizierungsverarbeitungsteil (11a) verarbeitet werden, wenn der Schaltungsteil (16a) geschlossen ist.

4. Biometrische Authentifizierungsvorrichtung (1) nach Anspruch 3, aufweisend:
eine Kommunikationsschnittstelle, die eingerichtet ist, mit einem externen Endgerät zu kommunizieren; und
einen Authentifizierungsdatenmeldeteil, der mit dem Schaltungsteil (16a) verbunden ist und eingerichtet ist, die biometrischen Authentifizierungsinformationen, die in dem Authentifizierungsdatenhalteteil (11b) gespeichert sind, über die Kommunikationsschnittstelle an das Endgerät zu übertragen, wenn der Schaltungsteil (16a) geschlossen ist.

5. Biometrische Authentifizierungsvorrichtung (1) nach einem der Ansprüche 2 bis 4, wobei der Betätigungsmechanismus (17) den biometrischen Authentifizierungssensor (12) so hält, dass er zwischen einer Ausgangsposition und einer Position, in der der Schalter betätigt wird, beweglich ist, und den biometrischen Authentifizierungssensor (12) in Richtung der Ausgangsposition vorspannt.

6. Biometrische Authentifizierungsvorrichtung (1) nach Anspruch 5, wobei der Betätigungsmechanismus (17) und der Schalter vom momentanen Typ sind.

7. Biometrische Authentifizierungsvorrichtung (1) nach Anspruch 5, wobei der Betätigungsmechanismus (17) oder der Schalter vom alternativen Typ sind.

8. Biometrische Authentifizierungsvorrichtung (1) nach einem der Ansprüche 1 bis 7, wobei der biometrische Authentifizierungssensor (12) ein Fingerabdrucksensor ist.

## Revendications

1. Dispositif d'authentification biométrique (1), comprenant :
un capteur d'authentification biométrique (12) configuré pour être amené en contact avec un doigt quelconque d'une main et pour acquérir des informations d'authentification biométrique à partir du doigt ;
un boîtier (19) logeant le capteur d'authentification biométrique (12), comprenant une ouverture (21) exposant le capteur d'authentification biométrique (12) à une partie d'une surface extérieure, et configuré pour être maintenu par les doigts de la main, le boîtier (19) ayant une forme cubique qui peut être ajustée dans une sphère ayant un diamètre de 7 cm ;
une batterie (15) disposée dans le boîtier (19) ; et
un circuit d'alimentation en puissance (16) configuré pour démarrer une alimentation en puissance à partir de la batterie (15) par une pression vers le bas du capteur d'authentification biométrique (12) dans un état dans lequel le doigt est en contact avec le capteur d'authentification biométrique (12) ;
dans lequel une rainure (22) est ménagée dans le boîtier (19), la rainure (22) étant ménagée dans une surface (19b) des quatre surfaces adjacente à une surface (19a), l'ouverture (21) étant située là où est exposé le capteur d'authentification biométrique (12).

2. Dispositif d'authentification biométrique (1) selon la revendication 1, dans lequel
le circuit d'alimentation en puissance (16) comprend une partie de circuit (16a) configurée pour connecter la batterie (15) et le capteur d'authentification biométrique (12), et un commutateur configuré pour ouvrir la partie de circuit (16a) et pour fermer la partie de circuit (16a) lorsqu'il est mis en œuvre, et
le dispositif d'authentification biométrique (1) comprend en outre un mécanisme de mise en œuvre (17) configuré pour mettre en œuvre le commutateur par une pression vers le bas du capteur d'authentification biométrique (12) dans un état dans lequel le doigt est en contact avec le capteur d'authentification biométrique (12).

3. Dispositif d'authentification biométrique (1) selon la revendication 2, comprenant :
une partie de traitement d'authentification (11a) connectée à la partie de circuit (16a), et configurée pour authentifier les informations d'authentification biométrique sur la base d'informations acquises par le capteur d'authentification biométrique (12) lorsque la partie de circuit (16a) est fermée ; et
une partie de maintien de données d'authentification (11b) connectée à la partie de circuit (16a), et configurée pour mémoriser les informations d'authentification biométrique traitées par la partie de traitement d'authentification (11a) lorsque la partie de circuit (16a) est fermée.

4. Dispositif d'authentification biométrique (1) selon la revendication 3, comprenant :
une interface de communication configurée pour communiquer avec un terminal externe ; et
une partie de rapport de données d'authentification connectée à la partie de circuit (16a), et configurée pour transmettre les informations d'authentification biométrique, mémorisées dans la partie de maintien de données d'authentification (11b), au terminal par le biais de l'interface de communication lorsque la partie de circuit (16a) est fermée.

5. Dispositif d'authentification biométrique (1) selon l'une quelconque des revendications 2 à 4, dans lequel le mécanisme de mise en œuvre (17) maintient le capteur d'authentification biométrique (12) pour qu'il soit mobile entre une position initiale et une position au niveau de laquelle le commutateur est mis en œuvre, et sollicite le capteur d'authentification biométrique (12) vers la position initiale.

6. Dispositif d'authentification biométrique (1) selon la revendication 5, dans lequel le mécanisme de mise en œuvre (17) et le commutateur font partie d'un système à rappel.

7. Dispositif d'authentification biométrique (1) selon la revendication 5, dans lequel le mécanisme de mise en œuvre (17) ou le commutateur fait partie d'un système à deux fonctions.

8. Dispositif d'authentification biométrique (1) selon l'une quelconque des revendications 1 à 7, dans lequel le capteur d'authentification biométrique (12) est un capteur d'empreintes digitales.
